# EUROPÄISCHE PATENTSCHRIFT

(11) **EP 0 608 714 B1**
(45) Veröffentlichungstag und Bekanntmachung des Hinweises auf die Patenterteilung: **02.05.1997**
(21) Anmeldenummer: 94100423.6
(22) Anmeldetag: 13.01.1994
(51) Int. Cl.: C07C 63/70, C07C 51/363

(54) **Verfahren zur Herstellung chlorierter 4,5-Difluorbenzoesäuren, -benzoesäurederivate und -benzaldehyde**
Process for preparing chlorinated 4,5-difluorobenzoic acids, -benzoic acid derivatives and -benzaldehyde
Procédé de préparation d'acides 4,5-difluorobenzoiques chlorés, dérivés d'acide -benzoique et de la -benzaldéhyde

(30) Priorität: 29.01.1993 DE 4302458
(43) Veröffentlichungstag der Anmeldung: 03.08.1994
(73) Patentinhaber: HOECHST AKTIENGESELLSCHAFT, 65929 Frankfurt am Main (DE)
(72) Erfinder: Pfirmann, Ralf, Dr., D-64347 Griesheim (DE); Papenfuhs, Theodor, Dr., D-60433 Frankfurt am Main (DE); Weichselbaumer, Georg, Dr., D-65779 Kelkheim (DE)

(56) Entgegenhaltungen:
- DE-A- 4 123 322
- PATENT ABSTRACTS OF JAPAN, vol. 14, no. 356 (C-745)(4299), 2. August 1990; & JP-A-02 131 447

## Beschreibung

Die vorliegende Erfindung betrifft ein neues und vorteilhaftes Verfahren zur Herstellung von in 2-Stellung und gegebenenfalls in 3-Stellung chlorierten 4,5-Difluorbenzoesäuren, -benzoesäurederivaten und -benzaldehyden. Die über das erfindungsgemäße Verfahren zugänglichen Verbindungen stellen wertvolle Zwischenprodukte für die Herstellung hochwirksamer antibakterieller Mittel dar.

2-Chlor-4,5-difluorbenzoesäure ist ein wichtiges Zwischenprodukt für die Synthese von Pharmazeutika aus der Reihe der Fluorchinoloncarbonsäuren. Die Herstellung dieser Wirkstoffe ist in EP-A-0 442 849, EP-A-0 321 191 und EP-A-0 303 291 beschrieben. Die Umsetzung von 2,3-Dichlor-4,5-difluorbenzoesäure zu Chinoloncarbonsäureantiinfektiva läßt sich beispielsweise aus der DE-OS 36 35 218 und EP-A-0 198 192 entnehmen.

Zur Herstellung von 2-Chlor-4,5-difluorbenzoesäure sind bislang nur unbefriedigende oder technisch nicht gangbare Synthesewege bekannt. So läßt sich gemäß EP-A-0 411 252 3,4-Difluorchlorbenzol mit Chloracetylchlorid oder Dichloracetylchlorid zu dem entsprechenden chlorierten Acetophenonderivat umsetzen, das mittels Chlorbleichlauge (Haloform-Reaktion) in die 2-Chlor-4,5-difluorbenzoesäure überführt werden kann. Die Verwendung von Chlorbleichlauge stellt einerseits einen nicht unerheblichen Kostenfaktor dar und führt andererseits zu einem beträchtlichen Anfall unerwünschter Abwässer. Darüberhinaus läßt sich das für die Herstellung von 3,4-Difluorchlorbenzol benötigte 1,2-Difluorbenzol nur mit hohem Aufwand durch Schiemann-Reaktion herstellen. Dies führt dazu, daß das Gesamtverfahren einen hohen technischen Aufwand erfordert und zudem unwirtschaftlich ist. Ein anderes Verfahren (EP-A-0 355 774) geht von 2,4-Dichlor-5-fluorbenzoesäureestern aus. Durch Halex-Reaktion (Chlor-Fluor-Austausch) der 2,4-Dichlor-5-fluorbenzoesäureester läßt sich zwar das 4-Chloratom mit relativ hoher Selektivität gegen Fluor austauschen, es bilden sich aber stets Isomere in einer Menge von 3 bis 8 %. Diese Isomeren lassen sich allerdings nur mit einem sehr hohen technischen Aufwand abtrennen. Zudem erhält man bei Einsatz von einfachen Estern lediglich geringe Ausbeuten, da diese Ester unter den Reaktionsbedingungen eine nur geringe Stabilität aufweisen. Lediglich bei Verwendung von sperrigen Estern oder Estern höherer Alkohole werden befriedigende bis gute Ausbeuten erzielt. Infolge der sterischen Hinderung wird jedoch die Hydrolyse dieser Ester zur Benzoesäure erschwert.

Als noch ungünstiger erweisen sich die bislang bekannten Verfahren zur Herstellung von 2,3-Dichlor-4,5-difluorbenzoesäure. Diese Verbindung ist aus EP-A-0 230 947 und EP-A-0 159 388 bekannt. Sie kann durch denitrierende Chlorierung oder über eine mehrstufige Synthese ausgehend von Benzonitrilen über bromierte Verbindungen hergestellt werden. Der mit einer derartigen mehrstufigen Synthese verbundene technische Aufwand ist sehr groß und führt zu hohen Fertigungskosten. Darüberhinaus sind, bedingt durch das mehrstufige Verfahren, die Gesamtausbeuten eher gering.

Das in der EP-A-0 230 947 beschriebene Verfahren erfordert sowohl die Verwendung von hochgiftigem Zyanid als auch den Einsatz von elementarem Brom zur Herstellung der bromierten Verbindungen. Eine der wichtigsten Anforderungen, die an moderne Pharmazeutika gerichtet werden, ist, daß sie bis in den Spurenbereich hinein frei von Brom sein müssen. Dies hat zur Folge, daß sowohl die Vorprodukte als auch die Zwischenprodukte weitestgehend bromfrei herzustellen sind.

Das in der EP-A-0 159 388 beschriebene Verfahren geht von Nitroverbindungen aus, die durch Austausch der Nitrogruppe mittels Chlor bei hohen Temperaturen in die gewünschten Produkte überführt werden. Nachteilig an diesem Verfahren ist, daß bereits die erforderlichen Ausgangsstoffe schwer herzustellen sind und überdies die Umsetzungen entsprechend den Reaktionsgleichgewichten nur unbefriedigend verlaufen, da die gewünschten Produkte wegen ihrer hohen Siedepunkte nicht aus der Reaktionsmischung abdestilliert werden können, wie dies im allgemeinen für glatte Reaktionsabläufe erforderlich ist, so daß diese Syntheseroute sich technisch nicht mit vertretbaren ökonomischen Aufwand realisieren läßt.

Daher bestand ein sehr hoher Bedarf nach einem neuen Verfahren zur Herstellung von in 2-Stellung und gegebenenfalls 3-Stellung chlorierten 4,5-Difluorbenzoesäuren, -benzoesäurederivaten und -benzaldehyden, das die beschriebenen Nachteile nicht aufweist und die gewünschten Verbindungen nicht nur mit relativ geringem technischen Aufwand, sondern auch in hoher Ausbeute und hoher Reinheit zugänglich macht.

Gelöst wird diese Aufgabe durch ein Verfahren zur Herstellung von Verbindungen der Formel 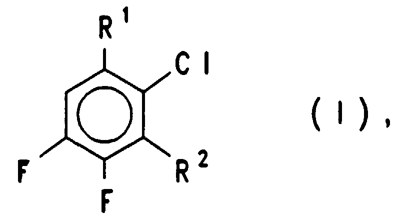 worin R¹ für -CN, -COOH, -COOR', wobei R' einen organischen Rest mit 1 bis 6 Kohlenstoffatomen darstellt, -CONH₂, -COCl oder -CHO und R² für H oder Cl steht. Es ist dadurch gekennzeichnet, daß man eine Verbindung der Formel 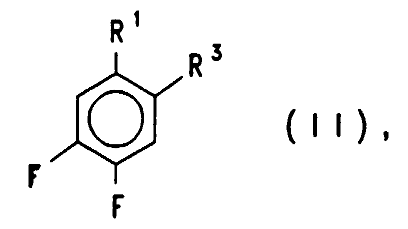 worin R¹ die obengenannte Bedeutung besitzt und R³ für H oder Cl steht, in Anwesenheit eines Chlorierungskatalysators mit einem Chlorierungsmittel bei -10 bis 200°C umsetzt.

Ein Vorteil ist, daß das erfindungsgemäße Verfahren sich nicht auf eine geringe Anzahl von Einsatzstoffen erstreckt, sondern sich bei einer Vielzahl verschiedener Verbindungen anwenden läßt. So eignen sich als Einsatzstoffe im allgemeinen Verbindungen der Formel (II) worin R¹ für -CN, -COOH, -COOR', wobei R' einen organischen Rest mit 1 bis 6 Kohlenstoffatomen, beispielsweise eine Methyl-, Ethyl-, Propyl, Isopropyl-, Butyl, Isobutyl- oder eine Pentyl- oder Hexylgruppe darstellt, -CONH₂, -COCl oder -CHO, insbesondere für -CN, -COOH, -COOR' oder -COCl, bevorzugt für -CN, -COOH oder -COCl, besonders bevorzugt für -CN oder -COOH
und R³ für H oder Cl steht.

Es ist auch möglich, Gemische von Verbindungen der allgemeinen Formel (II) in beliebiger Zusammensetzung zu verwenden. Hierunter fallen einerseits Gemische, in den Verbindungen mit R³ = H und R³ = Cl enthalten sind, und andererseits Mischungen, in denen die Verbindungen verschiedene Reste R¹ aufweisen, aber auch Kombinationen dieser beiden Mischungsvarianten.

Zum großen Teil leiten sich die vorstehend genannten Einsatzstoffe von der 3,4-Difluorbenzoesäure (entsprechend 4,5-Difluorbenzoesäure) ab und lassen sich aus dieser herstellen. Die 3,4-Difluorbenzoesäure kann zum einen durch Decarboxylierung von 4,5-Difluorphthalsäure, die ihrerseits ausgehend von 4,5-Dichlorphthalsäureanhydrid durch Chlor-Fluor-Austausch (Halex-Reaktion) und nachfolgende Hydrolyse des 4,5-Difluorphthalsäureanhydrids zugänglich ist (EP-A-0 055 630), hergestellt werden (EP-A-0 431 294). Alternativ ist es möglich, die beiden Fluoratome durch Halex-Reaktion in 3,4-Dichlorverbindungen einzuführen (N. Yazawa et al., Chem. Lett. (1989), 2212 bis 2216), wobei insbesondere 3,4-Dichlorbenzonitril, 3,4-Dichlorbenzoylchlorid oder 3,4-Dichlorbenzaldehyd in Betracht kommen.

Die entsprechenden 3,4-Difluorverbindungen können anschließend durch im Prinzip literaturkannte Methoden wie saure oder alkalische Hydrolyse in die entsprechende Säure überführt werden.

Durch das erfindungsgemäße Verfahren wird die als Einsatzstoff verwendete 3,4-Difluorverbindung (entsprechend 4,5-Difluorverbindung) durch Chlorierung zunächst in eine 2-Chlor-4,5-difluorverbindung umgewandelt und im Anschluß hieran unter Einführung eines weiteren Chloratoms in die entsprechende 2,3-Dichlor-4,5-difluorverbindung überführt. Je nachdem, welches Produkt hergestellt werden soll, kann die Umsetzung entweder nur bis zu der 2-Chlor-4,5-difluorverbindung, die gegebenenfalls zu der 2-Chlor-4,5-difluorbenzoesäure umgesetzt werden kann, oder weiter bis zu der 2,3-Dichlor-4,5-difluorverbindung, die gegebenenfalls zu der 2,3-Dichlor-4,5-difluorbenzoesäure verarbeitet werden kann, fortgeführt werden.

Die vorliegende Erfindung eröffnet ferner die Möglichkeit, sowohl die 2-Chlor-4,5-difluorverbindungen als auch die 2,3-Dichlor-4,5-difluorverbindungen herzustellen. Darüber hinaus gestattet sie es, durch Wahl der Verfahrensbedingungen die Bildung beider Produkte gezielt zu beeinflussen und das Verhältnis, mit dem die beiden Produkte gebildet werden, in relativ weiten Bereichen je nach Wunsch zu steuern. Auf diese Weise lassen sich von vorneherein Mischungen wunschgemäßer Zusammensetzung gezielt herstellen.

Eine Variante des erfindungsgemäßen Verfahrens besteht darin, daß man die Verbindung der Formel (II), worin R³ für H steht, mit dem Chlorierungsmittel zu der entsprechenden, in 2-Stellung chlorsubstituierten Verbindung umsetzt und diese Verbindung anschließend, gegebenenfalls nach Zwischenisolierung, insbesondere jedoch ohne Zwischenisolierung, mit einem Chlorierungsmittel zu der entsprechenden, in 2,3-Stellung dichlorierten Verbindung umsetzt.

Es ist allerdings auch möglich, nicht durch Chlorierung der entsprechenden 3,4-Difluorverbindung (respektive 4,5-Difluorverbindung) hergestellte 2-Chlor-4,5-difluorverbindungen (entsprechend der Formel (II), worin R³ für Cl steht) mit einem Chlorierungsmittel zu der entsprechenden in 2,3-Stellung dichlorierten Verbindung umzusetzen.

Es ist überraschend, daß bei der Chlorierung der 3,4-Difluorverbindungen zunächst ein Chloratom mit hoher Selektivität in die 2-Position eintritt, obwohl die dirigierenden Effekte der am Benzolkern vorhandenen Substituenten üblicherweise eine sogar recht selektive Substitution in der 3-Stellung erwarten lassen (J. March, Adv. Org. Chem. (1985, 3. edition) 16 bis 18, 453 bis 460). Ebenso überraschend ist es, daß in der zweiten Substitutionsstufe ein weiteres Chloratom wiederum mit hoher Selektivität in die 3-Stellung eintritt. Hierzu trägt möglicherweise neben dem in meta-Stellung dirigierenden Effekt der Carboxygruppe auch der durch das in die 2-Stellung eingeführte Chloratom ausgeübte, in die ortho-Stellung dirigierende Effekt bei. So entsteht in nicht zu erwartender Weise die entsprechende 2,3-Dichlor-4,5-difluorverbindung, insbesondere 2,3-Dichlor-4,5-difluorbenzoesäure, ohne daß infolge einer weiteren Chlorierung, selbst bei hohen Umsätzen, eine trichlorierte Verbindung oder andere Isomere in nennenswerter Menge entstehen.

Man führt die Umsetzung in Anwesenheit eines Chlorierungskatalysators durch. Geeignete Chlorierungskatalysatoren sind Jod, Jodtrichlorid, Jodpentachlorid, Eisen, Eisen(II)chlorid, Eisen(III)chlorid, Aluminiumchlorid, Dischwefeldichlorid, Antimontrichlorid und/oder Antimonpentachlorid, insbesondere Jod, Jodtrichlorid, Eisen(II)chlorid und/oder Dischwefeldichlorid, bevorzugt Jod und/oder Dischwefeldichlorid einsetzt. Es lassen sich jedoch auch Mischungen der vorstehend genannten Chlorierungskatalysatoren in beliebiger Zusammensetzung verwenden.

Der Chlorierungskatalysator wird in katalytischen Mengen, daß heißt nicht in stöchiometrischer Menge, bezogen auf die umzusetzende Verbindung, eingesetzt. Üblicherweise setzt man 0,001 bis 0,05, insbesondere 0,002 bis 0,02 Mol Chlorierungskatalysator je Mol zu chlorierende Verbindung ein.

Die Umsetzung wird unter Verwendung eines Chlorierungsmittels durchgeführt. Als Chlorierungsmittel sind Chlor und/oder ein chlorabgebendes Mittel geeignet. Ein besonders geeignetes Chlorierungsmittel ist Chlor.

Als chlorabgebende Mittel lassen sich Phosphortrichlorid, Phosphorpentachlorid, Antimonpentachlorid, Jodtrichlorid, Schwefeldichlorid, Dischwefeldichlorid und Mangantetrachlorid, insbesondere Phosphorpentachlorid und Jodtrichlorid, bevorzugt Phosphorpentachlorid verwenden.

Bei Durchführung der Umsetzung hat es sich in vielen Fällen als nützlich erwiesen, mit einer unter dem stöchiometrischen Bedarf liegenden Menge Chlor oder chlorabgebenden Mitteln zu arbeiten. Zweckmäßigerweise setzt man je Äquivalent zu substituierendem Wasserstoffatom 0,05 bis 0,95, insbesondere 0,1 bis 0,9, bevorzugt 0,25 bis 0,85 Mol Chlor oder chlorabgebendes Mittel ein. Für die Herstellung von 2-Chlor-4,5-difluorverbindungen aus 3,4-Difluorverbindungen setzt man in der Regel 0,1 bis 0,8, bevorzugt 0,25 bis 0,6 Mol Chlor oder chlorabgebendes Mittel je Mol 3,4-Difluorverbindung ein. Zur Umsetzung von 2-Chlor-4,5-difluorverbindungen zu den entsprechenden 2,3-Dichlor-4,5-difluorverbindungen werden im allgemeinen 0,3 bis 0,95, bevorzugt 0,5 bis 0,85 Mol Chlor oder chlorabgebendes Mittel je Mol 2-Chlor-4,5-difluorverbindung zugesetzt. Stellt man aus den 3,4-Difluorverbindungen die 2,3-Dichlor-4,5-difluorverbindungen her, so bringt man 0,3 bis 1,5, bevorzugt 0,7 bis 1,2 Mol Chlor bzw. chlorabgebendes Mittel je Mol 3,4-Difluorverbindung zur Umsetzung. Die tatsächlich erforderliche Menge Chlor (oder die sogenannte Chlorausbeute) ist in beträchtlichem Umfange auch von der Begasung, der Durchmischung der Reaktionsteilnehmer und der Art und Form der verwendeten Reaktionsapparate abhängig.

Es hat sich als zweckmäßig erwiesen, die Chlorströme oder bei Verwendung chlorabgebender Mittel, die Chlor-Freisetzungsraten so zu wählen, daß sie etwa 5 bis etwa 600, insbesondere etwa 20 bis etwa 400, bevorzugt etwa 40 bis etwa 200 ml, jeweils bezogen auf 1 g der zu chlorierenden Verbindung je Stunde, betragen.

Man arbeitet bei Temperaturen von -10 bis 200°C.
Nach einer besonderen Ausführungsform führt man die Umsetzung in Anwesenheit eines Lösungsmittels bei Temperaturen von -10 bis 80, insbesondere 0 bis 70, bevorzugt 10 bis 50°C durch. Als Lösungsmittel eignen sich Chlorsulfonsäure, Schwefelsäure oder Oleum, insbesondere Chlorsulfonsäure oder Oleum, bevorzugt Chlorsulfonsäure.

Mit guten Erfolg lassen sich auch Mischungen der vorstehend genannten Lösungsmittel, insbesondere Mischungen, die mindestens 10 Gew.-% Chlorsulfonsäure, insbesondere 10 bis 90 Gew.-% Chlorsulfonsäure enthalten, einsetzen.

Nach einer weiteren Ausführungsform des Verfahrens führt man die Umsetzung in Abwesenheit eines Lösungsmittels bei 100 bis 200, insbesondere 130 bis 180°C durch.

In einer Reihe von Fällen kann es sinnvoll sein, dem Chlorierungssumpf und insbesondere dem Wasser, das im allgemeinen zum Ausfällen des in den verwendeten Lösungsmitteln gelösten Produktes zugefügt wird, zur Verhinderung einer durch Fluorwasserstoff verursachten Korrosion, die bei derartigen Umsetzungen im erheblichen Maße auftreten kann, ein fluoridabfangendes Mittel zuzusetzen. Als fluoridabfangende Mittel kommen Calziumsalze wie Calziumchlorid, Calziumsulfat und Calziumhydroxid ebenso wie Kieselsäure, Kieselgele und Siliciumdioxid in Betracht. Man kann auch Mischungen der vorstehend erwähnten fluoridabfangenden Mittel verwenden.

Die gewünschten Wertprodukte der allgemeinen Formel (I) (hierzu zählen insbesondere 2,3-Dichlor-4,5-difluorbenzoesäure und 2-Chlor-4,5-difluorbenzoesäure und deren funktionelle Derivate) können beispielsweise durch Verdünnen der Reaktionsmischung mit Wasser und nachfolgende Filtration isoliert werden. Ihre weitere Reinigung erfolgt zweckmäßigerweise durch Umkristallisation, gegebenenfalls unter Zusatz von Aktivkohle. Es ist auch möglich, sie mittels Säulenchromatographie zu reinigen. Besonders einfach gestaltet sich die Reinigung, wenn man die Carbonsäure bzw. Carbonsäurederivate in die entsprechenden Carbonsäurechloride überführt und diese durch Fraktionierung destillativ trennt. Ein weiterer Vorteil dieser Methode besteht darin, daß die Säurechloride anschließend direkt zu den entsprechenden Wertprodukten weiterverarbeitet werden können. Die Säurechloride lassen sich nach den üblichen Methoden aus den im Rohgemisch enthaltenen Carbonsäuren herstellen.

Die Ausbeuten an 2,3-Dichlor-4,5-difluorbenzoesäure und 2-Chlor-4,5-difluorbenzoesäure betragen in der Regel 60 bis 90 %. Als Nebenprodukt entsteht in geringen Mengen 2,3,6-Trichlor-4,5-difluorbenzoesäure, die nach den beschriebenen Methoden abgetrennt und gegebenenfalls für andere Synthesen verwendet werden kann.

Die gegebenenfalls erfindungsgemäß hergestellten funktionellen Derivate der 2,3-Dichlor-4,5-difluorbenzoesäure und/oder der 2-Chlor-4,5-difluorbenzoesäure können gegebenenfalls nach den für die 3,4-Difluorverbindungen angegebenen, im Prinzip literaturbekannten Methoden (saure oder basische Hydrolyse) in die Säuren überführt werden.

Alle Verfahrensschritte können bei Atmosphärendruck, Unter- oder Überdruck durchgeführt werden. Die folgenden Beispiele erläutern das Verfahren.

### Beispiel 1

In 1900 g Chlorsulfonsäure werden 470 g (2,44 Mol) 2-Chlor-4,5-difluorbenzoesäure mit 14 g (55 mMol) Jod vorgelegt. Bei 30°C leitet man 6,5 h Chlor ein (11 bis 12 l/h). Die zu Beginn dunkelgrüne Mischung verfärbt sich während der leicht exothermen Reaktion nach braun. Danach wird unter Sole-Kühlung auf 3000 g Eis getropft, das ausgefallene, gelbliche Produkt abgesaugt und zweimal mit 500 g Wasser gewaschen. Man erhält 620 g feuchtes Produkt, das bei 45°C im Vakuum getrocknet wird (585 g trocken). Das trockene, angeschmolzene Produkt wird binnen 3 h bei 70°C mit 357 g (3 Mol) Thionylchlorid versetzt und 6 h bei 80°C gehalten. Danach wird das überschüssige Thionylchlorid (80 g) unter schwachem Vakuum (100 mbar) abgezogen. Die verbleibende, klare, leicht gelbliche Lösung wird im Vakuum fraktioniert destilliert. Dabei werden erhalten:
99,7 g (0,47 Mol, 19 %) 2-Chlor-4,5-difluorbenzoylchlorid (Sdp. 9 Torr/95°C)
381,5 g (1,55 Mol, 64 %) 2,3-Dichlor-4,5-difluorbenzoylchlorid (Sdp. 8 bis 9 Torr/106 bis 109°C)
51.6 g (0,185 Mol, 8 %) 2,3,6-Trichlor-4,5-difluorbenzoylchlorid (Sdp. 9 Torr/122 bis 124°C)

Die wäßrige Mutterlauge wird mit Methyl-tert.-butylether (MTBE) extrahiert. nach Trocknen der organischen Phase und Entfernen des Lösungsmittels erhält man 32,0 g gelben Feststoff, der nach gaschromatografischer Analyse (GC) 26,7 g (0,14 Mol, 6 %) 2-Chlor-4,5-difluorbenzoesäure enthält.
2,3-Dichlor-4,5-difluorbenzoesäure:
¹⁹F-NMR [DMSO-d₆ ppm, CFCl₃]:
δ = -127,72 (dd, 1F, J_{AB} = 8,1 Hz, J_{BC} = 22,8 Hz, Ar-F⁴(B)) -134,76 (dd, 1F, J_{AC} = 10,2 Hz, J_{BC} = 22,2 Hz, Ar-F⁵ (C))

### Beispiel 2

In 500 g Oleum werden 100 g (0,52 Mol) 2-Chlor-4,5-difluorbenzoesäure mit 1 g Jod vorgelegt. Man leitet bei 35 bis 50°C 2,5 h Chlor ein (8 bis 10 l/h). Nach beendeter Chlorierung wird auf 400 g Eis gegeben und der ausgefallene Feststoff abfiltriert. Man erhält 286 g feuchtes Produkt, das mit Thionylchlorid (2 Mol) bei 80°C zu einer Mischung von Säurechloriden umsetzt wird. Diese werden, wie in Beispiel 1 beschrieben, durch fraktionierte Destillation getrennt. Man erhält 7 g (33 mMol, 6 %) 2-Chlor-4,5-difluorbenzoylchlord, 76 g (0,31 Mol, 60 %) 2,3-Dichlor-4,5-difluorbenzoylchlorid und 29,8 g (0,11 Mol, 20 %) 2,3,6-Trichlor-4,5-difluorbenzoylchlorid. Die Siedepunkte verhalten sich wie in Beispiel 1 beschrieben. Zur Reinigung der Hauptfraktion wird diese mit Wasser hydrolysiert und die erhaltene 2,3-Dichlor-4,5-difluorbenzoesäure aus heißem Wasser umkristallisiert. Man erhält diese als farblose Kristalle (68,5 g) vom Schmelzpunkt 130,5°C (DSC).

### Beispiel 3

In 200 g Oleum werden 1,5 g Eisen(III)chlorid und 31,5 g (0,2 Mol) 3,4-Difluorbenzoesäure bei 25 bis 35°C vorgelegt. Man leitet 7 l/h Chlor binnen 4 h ein und verdünnt unter guter Kühlung mit 100 g Wasser. Man erhält 33 g feuchte Mischung aus Benzoesäuren, die Mutterlauge wird verworfen. Man setzt bei 80°C mit Thionylchlorid um und destilliert die flüchtigen Bestandteile ab (37 g). Nach gaschromatographischer Analyse besteht die Mischung aus 11,2 g (64 mMol, 32 %) 3,4-Difluorbenzoylchlorid, 14,7 g (70 mMol, 35 %) 2-Chlor-4,5-difluorbenzoylchlorid und 10,9 g (44 mMol, 22 %) 2,3-Dichlor-4,5-difluorbenzoylchlorid. Die Mischung wird zusammen mit den Rohmischungen mehrerer analoger Ansätze durch Fraktionierung, wie in Beispiel 1 beschrieben, gereinigt. Das 3,4-Difluorbenzoylchlorid geht bei 9 Torr/79 bis 80° über.

### Beispiel 4

In 100 g Oleum/100 % Schwefelsäure (1:1) werden 0,5 g Dischwefeldichlorid vorgelegt und 10,9 g (69 mMol) 3,4-Difluorbenzoesäure eingetragen. Man chloriert zwischen -5 und 0°C (6 h) und verdünnt anschließend mit 100 g Eis. Das feuchte Produkt wird abfiltriert (15,2 g), man erhält nach Trocknen 13,8 g Rohprodukt. Dieses wird nach dem in Beispiel 1 beschriebenen Verfahren in die Säurechloride überführt, die durch Fraktionierung, zusammen mit anderen Ansätzen getrennt wurden. Durch quantitative GC-Analyse wird in der Mischung 3,3 g (19 mMol, 27 %) 3,4-Difluorbenzoylchlorid, 7,2 g (34 mMol, 50 %) 2-Chlor-4,5-difluorbenzoylchlorid und 2,6 g (11 mMol, 15 %) 2,3-Dichlor-4,5-difluorbenzyolchlorid nachgewiesen.

### Beispiel 5

15,8 g (0,1 Mol) 3,4-Difluorbenzoesäure werden in 50 g Chlorsulfonsäure und 50 g 100 %iger Schwefelsäure unter Anwesenheit von 0,5 g Eisenchlorid bei 40°C 2 h chloriert (1 l/h). Nach dieser Zeit wird das Chlor ausgeblasen und mit 50 g Eis versetzt. Man filtriert die Mischung der Carbonsäuren ab (16,0 g trocken) und trennt die Produkte durch fraktionierte Kristallisation aus Wasser bzw. Hexan, wobei teilweise durch Restlöslichkeit im Wasser verbliebene Produkte durch Extraktion mit MTBE wieder recyclisiert werden. Man erhält 2,3 g (14,5 mMol, 15 %) 3,4-Difluorbenzoesäure zurück, 8,1 g (42 mMol, 42 %) 2-Chlor-4,5-difluorbenzoesäure und 4,3 g (19 mMol, 19 %) 2,3-Dichlor-4,5-difluorbenzoesäure, jeweils mit Reingehalten > 95 % als farblose bis leicht gelbliche Feststoffe.

### Beispiel 6

In 1000 g Chlorsulfonsäure werden 3 g Jod und 2 g Dischwefeldichlorid vorgelegt. Dazu trägt man bei 0°C 139,1 g (1 Mol) 3,4-Difluorbenzonitril ein. Man beginnt Chlor einzuleiten (10 l/h) und steigert die Temperatur binnen 30 min auf 30°C. Nach 2,5 h wird die Chlor-Zufuhr abgestellt und überschüssiges, gelöstes Chlor ausgeblasen. Man versetzt unter guter Kühlung tropfenweise mit 500 g Wasser und erhitzt die gesamte Mischung 2 h auf 140°C (Hydrolyse der intermediär entstandenen Benzamide und der Nitrile). Nach dieser Zeit wird weiter gekühlt und mit weiteren 800 g Wasser verdünnt. Der ausfallende Feststoff wird abgesaugt (245 g feucht, 190 g trocken) und mit Thionylchlorid (2 Mol) bei 80°C umgesetzt. Die Auftrennung der Säurechloride erfolgt durch Fraktionierung, man erhält als farblose Flüssigkeiten:
33,3 g (0,19 Mol, 19 %) 3,4-Difluorbenzoylchlorid (Sdp. 9 Torr/79°C)
97,8 g (0,46 Mol, 46 %) 2-Chlor-4,5-difluorbenzoylchlorid (Sdp. 9 Torr/95°C)
71.2 g (0,29 Mol, 29 %) 2,3-Dichlor-4,5-difluorbenzoylchlorid (Sdp. 8 bis 9 Torr/107 bis 108°C)
2,9 g (10 mMol, 1 %) 2,3,6-Trichlor-4,5-difluorbenzoylchlorid (Sdp. 9 Torr/122 bis 124°C)

Setzt man anstatt 139,1 g (1 Mol) 3,4-Difluorbenzonitril 157, 1 g (1 Mol) 3,4-Difluorbenzamid ein und verfährt ansonsten, wie angegeben, so erhält man im wesentlichen dasselbe Ergebnis.

### Beispiel 7

In 500 g Chlorsulfonsäure werden 211 g (1 Mol) 2-Chlor-4,5-difluorbenzoylchlorid eingetragen und 3 g Jod zugefügt. Man chloriert (10 l/h) 1,7 h bei 40°C und verdünnt die Mischung anschließend unter Rühren mit Wasser (500 g), wobei man die Temperatur auf 80°C steigen läßt. Die zunächst im Lösungsmittel enthaltenen Säurechloride werden dabei in 2 h zu den Carbonsäuren hydrolysiert, die man nach Abkühlen auf 0°C durch Filtration isoliert. Die Mischung wird nach dem Trocknen mit Thionylchlorid (2 Mol) bei 80°C umgesetzt und überschüssiges Thionylchlorid anschließend abdestilliert. Danach wird die Mischung, wie in Beispiel 1 angegeben, destilliert. Man erhält 46,4 g (0,22 Mol, 22 %) 2-Chlor-4,5-difluorbenzoylchlorid und 174,9 g (0,71 Mol, 71 %) 2,3-Dichlor-4,5-difluorbenzoylchlorid.

## Patentansprüche

1. Verfahren zur Herstellung von Verbindungen der Formel 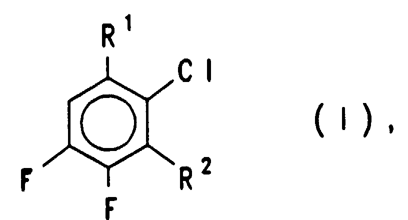 worin R¹ für -CN, -COOH, -COOR', wobei R' einen organischen Rest mit 1 bis 6 Kohlenstoffatomen darstellt, -CONH₂, -COCl oder -CHO und R² für H oder Cl steht, dadurch gekennzeichnet, daß man eine Verbindung der Formel 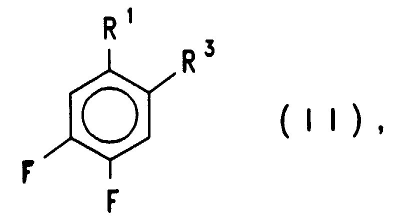 worin R¹ die obengenannte Bedeutung besitzt und R³ für H oder Cl steht, in Anwesenheit eines Chlorierungskatalysators mit einem Chlorierungsmittel bei -10 bis 200°C umsetzt.

2. Verfahren nach Anspruch 1, dadurch gekennzeichnet, daß man die Verbindung 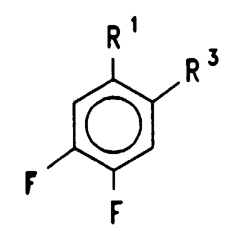 worin R³ für H steht, mit einem Chlorierungsmittel zu der Verbindung 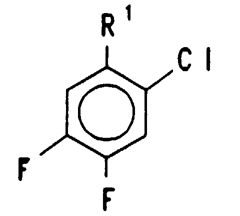 umsetzt und diese Verbindung anschließend mit einem Chlorierungsmittel zu der Verbindung 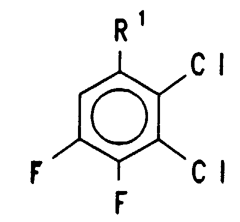 umsetzt.

3. Verfahren nach Anspruch 1, dadurch gekennzeichnet, daß man die Verbindung 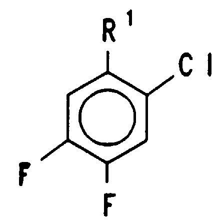 mit einem Chlorierungsmittel zu der Verbindung 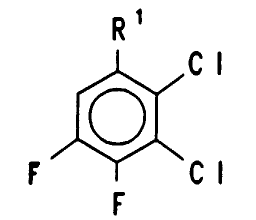 umsetzt.

4. Verfahren nach einem oder mehreren der Ansprüche 1 bis 3, dadurch gekennzeichnet, daß man als Chlorierungskatalysator Jod, Jodtrichlorid, Jodpentachlorid, Eisen, Eisen(II)chlorid, Eisen(III)chlorid, Aluminiumchlorid, Dischwefeldichlorid, Antimontrichlorid und/oder Antimonpentachlorid einsetzt.

5. Verfahren nach einem oder mehreren der Ansprüche 1 bis 4, dadurch gekennzeichnet, daß man 0,001 bis 0,05 insbesondere 0,002 bis 0,02 Mol Chlorierungskatalysator je Mol zu chlorierende Verbindung einsetzt.

6. Verfahren nach einem oder mehreren der Ansprüche 1 bis 5, dadurch gekennzeichnet, daß man als Chlorierungsmittel Chlor und/oder ein chlorabgebendes Mittel einsetzt.

7. Verfahren nach einem oder mehreren der Ansprüche 1 bis 6, dadurch gekennzeichnet, daß man als Chlorierungsmittel Chlor einsetzt.

8. Verfahren nach einem oder mehreren der Ansprüche 1 bis 6, dadurch gekennzeichnet, daß man als chlorabgebendes Mittel Phosphortrichlorid, Phosphorpentachlorid, Antimonpentachlorid, Jodtrichlorid, Schwefeldichlorid, Dischwefeldichlorid, Mangantetrachlorid, insbesondere Phosphorpentachlorid einsetzt.

9. Verfahren nach einem oder mehreren der Ansprüche 1 bis 8, dadurch gekennzeichnet, daß man je Äquivalent zu substituierenden H 0,05 bis 0,95, insbesondere 0,1 bis 0,9, bevorzugt 0,25 bis 0,85 Mol Chlor oder chlorabgebendes Mittel einsetzt.

10. Verfahren nach einem oder mehreren der Ansprüche 1 bis 9, dadurch gekennzeichnet, daß man die Umsetzung in Anwesenheit eines Lösungsmittels bei Temperaturen von -10 bis 80, insbesondere 0 bis 70, bevorzugt 10 bis 50°C durchführt.

11. Verfahren nach Anspruch 10, dadurch gekennzeichnet, daß man als Lösungsmittel Chlorsulfonsäure, Schwefelsäure und/oder Oleum, insbesondere Chlorsulfonsäure einsetzt.

12. Verfahren nach einem oder mehreren der Ansprüche 1 bis 9, dadurch gekennzeichnet, daß man die Umsetzung in Abwesenheit eines Lösungsmittels bei 100 bis 200, insbesondere 130 bis 180°C durchführt.

## Claims

1. A process for the preparation of compounds of the formula 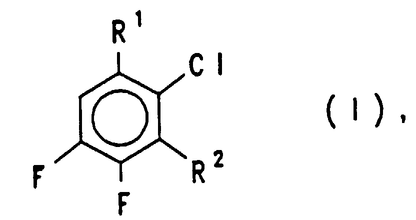 in which R¹ is -CN, -COOH, -COOR', R' being an organic radical having 1 to 6 carbon atoms, -CONH₂, -COCl or -CHO and R² is H or Cl, which comprises reacting a compound of the formula 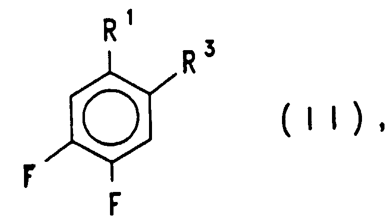 in which R¹ is as defined above and R³ is H or Cl, with a chlorinating agent in the presence of a chlorination catalyst at -10 to 200°C.

2. The process as claimed in claim 1, wherein the compound 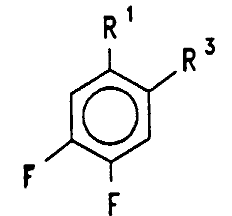 in which R³ is H, is reacted with a chlorinating agent to give the compound 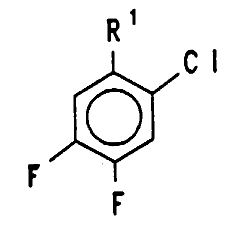 and this compound is then reacted with a chlorinating agent to give the compound

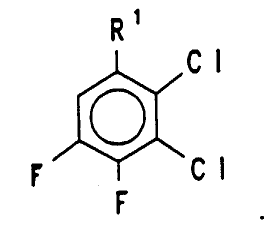

3. The process as claimed in claim 1, wherein the compound 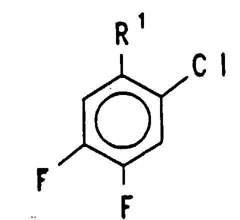 is reacted with a chlorinating agent to give the compound

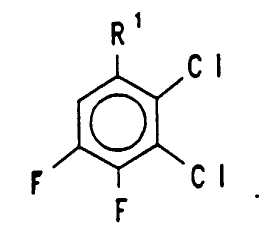

4. The process as claimed in one or more of claims 1 to 3, wherein the chlorination catalyst used is iodine, iodine trichloride, iodine pentachloride, iron, iron(II) chloride, iron(III) chloride, aluminum chloride, disulfur dichloride, antimony trichloride and/or antimony pentachloride.

5. The process as claimed in one or more of claims 1 to 4, wherein 0.001 to 0.05, especially 0.002 to 0.02, mol of chlorination catalyst is used per mole of compound to be chlorinated.

6. The process as claimed in one or more of claims 1 to 5, wherein the chlorinating agent used is chlorine and/or a chlorine-releasing agent.

7. The process as claimed in one or more of claims 1 to 6, wherein the chlorinating agent used is chlorine.

8. The process as claimed in one or more of claims 1 to 6, wherein the chlorine-releasing agent used is phosphorus trichloride, phosphorus pentachloride, antimony pentachloride, iodine trichloride, sulfur dichloride, disulfur dichloride or manganese tetrachloride, especially phosphorus pentachloride.

9. The process as claimed in one or more of claims 1 to 8, wherein 0.05 to 0.95, especially 0.1 to 0.9 and preferably 0.25 to 0.85, mol of chlorine or chlorine-releasing agent is used per equivalent of H to be substituted.

10. The process as claimed in one or more of claims 1 to 9, wherein the reaction is carried out in the presence of a solvent at temperatures from -10 to 80, especially 0 to 70 and preferably 10 to 50, °C.

11. The process as claimed in claim 10, wherein the solvent used is chlorosulfonic acid, sulfuric acid and/or oleum, especially chlorosulfonic acid.

12. The process as claimed in one or more of claims 1 to 9, wherein the reaction is carried out in the absence of a solvent at 100 to 200, especially 130 to 180, °C.

## Revendications

1. Procédé de préparation de composés de formule 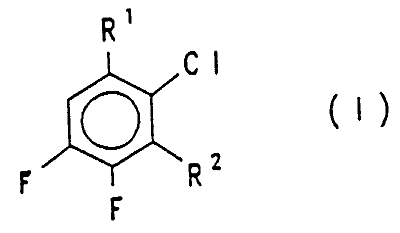 dans laquelle R¹ représente -CN, -COOH, -COOR', R' représentant un radical organique avec 1 à 6 atomes de carbone, -CONH₂, -COCl ou -CHO et R² représente H ou Cl, caractérisé en ce qu'on fait réagir un composé de formule 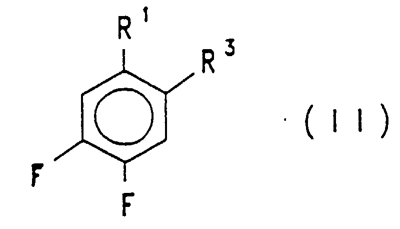 dans laquelle R¹ possède la signification indiquée ci-dessus et R³ représente H ou Cl, en présence d'un catalyseur de chloration, avec un agent de chloration à une température de -10 à 200°C.

2. Procédé selon le revendication 1, caractérisé en ce qu'on fait réagir le composé 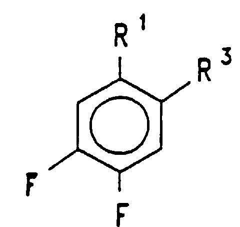 dans lequel R³ représente H, avec un agent de chloration pour obtenir le composé 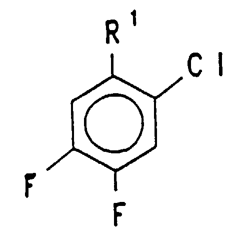 et en ce qu'on transforme ensuite ce composé, au moyen d'un agent de chloration, en le composé

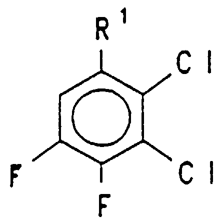

3. Procédé selon la revendication 1, caractérisé en ce qu'on fait réagir-le composé 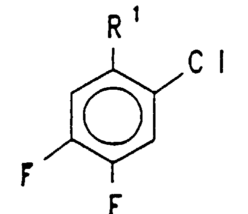 avec un agent de chloration, pour obtenir le composé

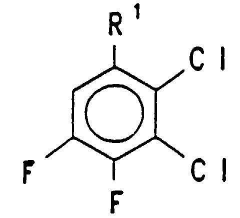

4. procédé selon l'une ou plusieurs des revendications 1 à 3, caractérisé en ce qu'on utilise, comme catalyseur de chloration, l'iode, le trichlorure d'iode, le pentachlorure d'iode, le fer, le chlorure de fer(II), le chlorure de fer(III), le chlorure d'aluminium, le chlorure de soufre, le trichlorure d'antimoine et/ou le pentachlorure d'antimoine.

5. Procédé selon l'une ou plusieurs des revendications 1 à 4 , caractérisé en ce qu'on utilise 0,001 à 0,05, en particulier 0,002 à 0,02 mol de catalyseur de chloration par mol de composé à chlorer.

6. Procédé selon l'une ou plusieurs des revendications 1 à 5, caractérisé en ce qu'on utilise, comme agent de chloration, le chlore et/ou un agent libérant du chlore.

7. Procédé selon l'une ou plusieurs des revendications 1 à 6, caractérisé en ce qu'on utilise du chlore comme agent de chloration.

8. Procédé selon l'une ou plusieurs des revendications 1 à 6, caractérisé en ce qu'on utilise, comme agent libérant du chlore, le trichlorure de phosphore, le pentachlorure de phosphore, le pentachlorure d'antimoine, le trichlorure d'iode, le bichlorure de soufre, le chlorure de soufre, le tétrachlorure de manganèse, en particulier le pentachlorure de phosphore.

9. Procédé selon l'une ou plusieurs des revendications 1 à 8, caractérisé en ce qu'on utilise, par équivalent d'atome d'hydrogène à substituer, 0,05 à 0,95, en particulier 0,1 à 0,9, de préférence 0,25 à 0,85 mol de chlore ou d'agent libérant du chlore.

10. Procédé selon l'une ou plusieurs des revendications 1 à 9, caractérisé en ce qu'on effectue la réaction en présence d'un solvant à des températures de -10 à 80, en particulier de 0 à 70, de préférence de 10 à 50°C.

11. Procédé selon la revendication 10, caractérisé en ce qu'on utilise comme solvant de l'acide chlorosulfonique, de l'acide sulfurique et/ou de l'oléum, en particulier de l'acide chlorosulfonique.

12. Procédé selon l'une ou plusieurs des revendications 1 à 9, caractérisé en ce qu'on effectue la réaction en l'absence d'un solvant à 100 jusqu'à 200, en particulier 130 jusqu'à 180°C.
